# EUROPEAN PATENT APPLICATION

(11) **EP 3 907 282 A1**
(43) Date of publication of application: **10.11.2021**
(21) Application number: 20178779.3
(22) Date of filing: 08.06.2020
(51) Int. Cl.: C12N 7/02, A61K 39/215

(54) **PREPARATION OF VIRUS-LIKE PARTICLES FOR ACCURATE ANALYSIS AND IMMUNIZATION AGAINST CORONA VIRUSES**

(30) Priority: 08.05.2020 EP 20173772
(71) Applicant: ETH Zurich, 8092 Zürich (CH)
(72) Inventor: Stark, Wendelin Jan, 4900 Langenthal (CH); Christen, Matthias, 4153 Reinach (CH); Christen, Beat, 5408 Ennetbaden (CH); Kobert, Nikita, 8038 Zürich (CH); Lüscher, Anne, 8052 Zürich (CH); Gröninger, Olivier, 5400 Baden (CH); Schächle, Philipp, 9492 Eschen (LI)
(74) Representative: Rüedi, Regula Béatrice

(57) **Abstract**

An avirulent virus-like particle comprising the spike protein S of a target corona virus and at least two and preferably 3 shell-forming proteins being further membrane proteins of corona viruses, said shell-forming proteins being the structural envelope protein E and the membrane protein M and optionally and preferably also the nucle-oprotein N, said virus-like particle comprising a protein shell and a glycosylation pattern obtainable by expression in mammalian cells is described as well as its use as analytical agent and as active ingredient in vaccines.

SEQUENCE LISTING

<110> ETH Zürich

<120> Preparation of virus-like particles for accurate analysis and
immunization against Corona viruses

<130> P183219EP01

<150> EP20 173 772.3
<151> 2020-05-08

<160> 4

<170> BiSSAP 1.3.6

<210> 1
<211> 76
<212> PRT
<213> viruses

<220>
<223> Synthetic envelope protein E of Sars CoV 2

<400> 1
Met Val Tyr Ser Phe Val Ser Glu Glu Thr Gly Thr Leu Ile Val Asn
1               5                   10                  15
Ser Val Leu Leu Phe Leu Ala Phe Val Val Phe Leu Leu Val Thr Leu
            20                  25                  30
Ala Ile Leu Thr Ala Leu Arg Leu Cys Ala Tyr Cys Cys Asn Ile Val
        35                  40                  45
Asn Val Ser Leu Val Lys Pro Ser Phe Tyr Val Tyr Ser Arg Val Lys
    50                  55                  60
Asn Leu Asn Ser Ser Arg Val Pro Asp Leu Leu Val
65                  70                  75

<210> 2
<211> 223
<212> PRT
<213> viruses

<220>
<223> Synthetic membrane protein M of Sars CoV 2

<400> 2
Met Val Ala Asp Ser Asn Gly Thr Ile Thr Val Glu Glu Leu Lys Lys
1               5                   10                  15
Leu Leu Glu Gln Trp Asn Leu Val Ile Gly Phe Leu Phe Leu Thr Trp
            20                  25                  30
Ile Cys Leu Leu Gln Phe Ala Tyr Ala Asn Arg Asn Arg Phe Leu Tyr
        35                  40                  45
Ile Ile Lys Leu Ile Phe Leu Trp Leu Leu Trp Pro Val Thr Leu Ala
    50                  55                  60
Cys Phe Val Leu Ala Ala Val Tyr Arg Ile Asn Trp Ile Thr Gly Gly
65                  70                  75                  80
Ile Ala Ile Ala Met Ala Cys Leu Val Gly Leu Met Trp Leu Ser Tyr
                85                  90                  95
Phe Ile Ala Ser Phe Arg Leu Phe Ala Arg Thr Arg Ser Met Trp Ser
            100                 105                 110
Phe Asn Pro Glu Thr Asn Ile Leu Leu Asn Val Pro Leu His Gly Thr
        115                 120                 125

Ile Leu Thr Arg Pro Leu Leu Glu Ser Glu Leu Val Ile Gly Ala Val
    130                 135                 140
Ile Leu Arg Gly His Leu Arg Ile Ala Gly His His Leu Gly Arg Cys
145                 150                 155                 160
Asp Ile Lys Asp Leu Pro Lys Glu Ile Thr Val Ala Thr Ser Arg Thr
                165                 170                 175
Leu Ser Tyr Tyr Lys Leu Gly Ala Ser Gln Arg Val Ala Gly Asp Ser
            180                 185                 190
Gly Phe Ala Ala Tyr Ser Arg Tyr Arg Ile Gly Asn Tyr Lys Leu Asn
        195                 200                 205
Thr Asp His Ser Ser Ser Ser Asp Asn Ile Ala Leu Leu Val Gln
    210                 215                 220

<210> 3
<211> 420
<212> PRT
<213> viruses

<220>
<223> Synthetic nucleoprotein N of Sars CoV 2

<400> 3
Met Val Ser Asp Asn Gly Pro Gln Asn Gln Arg Asn Ala Pro Arg Ile
1               5                   10                  15
Thr Phe Gly Gly Pro Ser Asp Ser Thr Gly Ser Asn Gln Asn Gly Glu
            20                  25                  30
Arg Ser Gly Ala Arg Ser Lys Gln Arg Arg Pro Gln Gly Leu Pro Asn
        35                  40                  45
Asn Thr Ala Ser Trp Phe Thr Ala Leu Thr Gln His Gly Lys Glu Asp
    50                  55                  60
Leu Lys Phe Pro Arg Gly Gln Gly Val Pro Ile Asn Thr Asn Ser Ser
65                  70                  75                  80
Pro Asp Asp Gln Ile Gly Tyr Tyr Arg Arg Ala Thr Arg Arg Ile Arg
                85                  90                  95
Gly Gly Asp Gly Lys Met Lys Asp Leu Ser Pro Arg Trp Tyr Phe Tyr
            100                 105                 110
Tyr Leu Gly Thr Gly Pro Glu Ala Gly Leu Pro Tyr Gly Ala Asn Lys
        115                 120                 125
Asp Gly Ile Ile Trp Val Ala Thr Glu Gly Ala Leu Asn Thr Pro Lys
    130                 135                 140
Asp His Ile Gly Thr Arg Asn Pro Ala Asn Asn Ala Ala Ile Val Leu
145                 150                 155                 160
Gln Leu Pro Gln Gly Thr Thr Leu Pro Lys Gly Phe Tyr Ala Glu Gly
                165                 170                 175
Ser Arg Gly Gly Ser Gln Ala Ser Ser Arg Ser Ser Ser Arg Ser Arg
            180                 185                 190
Asn Ser Ser Arg Asn Ser Thr Pro Gly Ser Ser Arg Gly Thr Ser Pro
        195                 200                 205
Ala Arg Met Ala Gly Asn Gly Gly Asp Ala Ala Leu Ala Leu Leu Leu
    210                 215                 220
Leu Asp Arg Leu Asn Gln Leu Glu Ser Lys Met Ser Gly Lys Gly Gln
225                 230                 235                 240
Gln Gln Gln Gly Gln Thr Val Thr Lys Lys Ser Ala Ala Glu Ala Ser
                245                 250                 255
Lys Lys Pro Arg Gln Lys Arg Thr Ala Thr Lys Ala Tyr Asn Val Thr
            260                 265                 270
Gln Ala Phe Gly Arg Arg Gly Pro Glu Gln Thr Gln Gly Asn Phe Gly
        275                 280                 285
Asp Gln Glu Leu Ile Arg Gln Gly Thr Asp Tyr Lys His Trp Pro Gln
    290                 295                 300
Ile Ala Gln Phe Ala Pro Ser Ala Ser Ala Phe Phe Gly Met Ser Arg
305                 310                 315                 320

Ile Gly Met Glu Val Thr Pro Ser Gly Thr Trp Leu Thr Tyr Thr Gly
                325                 330                 335
Ala Ile Lys Leu Asp Asp Lys Asp Pro Asn Phe Lys Asp Gln Val Ile
            340                 345                 350
Leu Leu Asn Lys His Ile Asp Ala Tyr Lys Thr Phe Pro Pro Thr Glu
        355                 360                 365
Pro Lys Lys Asp Lys Lys Lys Lys Ala Asp Glu Thr Gln Ala Leu Pro
    370                 375                 380
Gln Arg Gln Lys Lys Gln Gln Thr Val Thr Leu Leu Pro Ala Ala Asp
385                 390                 395                 400
Leu Asp Asp Phe Ser Lys Gln Leu Gln Gln Ser Met Ser Ser Ala Asp
                405                 410                 415
Ser Thr Gln Ala
            420

<210> 4
<211> 1274
<212> PRT
<213> viruses

<220>
<223> Synthetic surface protein S of Sars CoV 2

<400> 4
Met Val Phe Val Phe Leu Val Leu Leu Pro Leu Val Ser Ser Gln Cys
1               5                   10                  15
Val Asn Leu Thr Thr Arg Thr Gln Leu Pro Pro Ala Tyr Thr Asn Ser
            20                  25                  30
Phe Thr Arg Gly Val Tyr Tyr Pro Asp Lys Val Phe Arg Ser Ser Val
        35                  40                  45
Leu His Ser Thr Gln Asp Leu Phe Leu Pro Phe Phe Ser Asn Val Thr
    50                  55                  60
Trp Phe His Ala Ile His Val Ser Gly Thr Asn Gly Thr Lys Arg Phe
65                  70                  75                  80
Asp Asn Pro Val Leu Pro Phe Asn Asp Gly Val Tyr Phe Ala Ser Thr
                85                  90                  95
Glu Lys Ser Asn Ile Ile Arg Gly Trp Ile Phe Gly Thr Thr Leu Asp
            100                 105                 110
Ser Lys Thr Gln Ser Leu Leu Ile Val Asn Asn Ala Thr Asn Val Val
        115                 120                 125
Ile Lys Val Cys Glu Phe Gln Phe Cys Asn Asp Pro Phe Leu Gly Val
    130                 135                 140
Tyr Tyr His Lys Asn Asn Lys Ser Trp Met Glu Ser Glu Phe Arg Val
145                 150                 155                 160
Tyr Ser Ser Ala Asn Asn Cys Thr Phe Glu Tyr Val Ser Gln Pro Phe
                165                 170                 175
Leu Met Asp Leu Glu Gly Lys Gln Gly Asn Phe Lys Asn Leu Arg Glu
            180                 185                 190
Phe Val Phe Lys Asn Ile Asp Gly Tyr Phe Lys Ile Tyr Ser Lys His
        195                 200                 205
Thr Pro Ile Asn Leu Val Arg Asp Leu Pro Gln Gly Phe Ser Ala Leu
    210                 215                 220
Glu Pro Leu Val Asp Leu Pro Ile Gly Ile Asn Ile Thr Arg Phe Gln
225                 230                 235                 240
Thr Leu Leu Ala Leu His Arg Ser Tyr Leu Thr Pro Gly Asp Ser Ser
                245                 250                 255
Ser Gly Trp Thr Ala Gly Ala Ala Ala Tyr Tyr Val Gly Tyr Leu Gln
            260                 265                 270
Pro Arg Thr Phe Leu Leu Lys Tyr Asn Glu Asn Gly Thr Ile Thr Asp
        275                 280                 285
Ala Val Asp Cys Ala Leu Asp Pro Leu Ser Glu Thr Lys Cys Thr Leu
    290                 295                 300

Lys Ser Phe Thr Val Glu Lys Gly Ile Tyr Gln Thr Ser Asn Phe Arg
305                 310                 315                 320
Val Gln Pro Thr Glu Ser Ile Val Arg Phe Pro Asn Ile Thr Asn Leu
                325                 330                 335
Cys Pro Phe Gly Glu Val Phe Asn Ala Thr Arg Phe Ala Ser Val Tyr
            340                 345                 350
Ala Trp Asn Arg Lys Arg Ile Ser Asn Cys Val Ala Asp Tyr Ser Val
        355                 360                 365
Leu Tyr Asn Ser Ala Ser Phe Ser Thr Phe Lys Cys Tyr Gly Val Ser
    370                 375                 380
Pro Thr Lys Leu Asn Asp Leu Cys Phe Thr Asn Val Tyr Ala Asp Ser
385                 390                 395                 400
Phe Val Ile Arg Gly Asp Glu Val Arg Gln Ile Ala Pro Gly Gln Thr
                405                 410                 415
Gly Lys Ile Ala Asp Tyr Asn Tyr Lys Leu Pro Asp Asp Phe Thr Gly
            420                 425                 430
Cys Val Ile Ala Trp Asn Ser Asn Asn Leu Asp Ser Lys Val Gly Gly
        435                 440                 445
Asn Tyr Asn Tyr Leu Tyr Arg Leu Phe Arg Lys Ser Asn Leu Lys Pro
    450                 455                 460
Phe Glu Arg Asp Ile Ser Thr Glu Ile Tyr Gln Ala Gly Ser Thr Pro
465                 470                 475                 480
Cys Asn Gly Val Glu Gly Phe Asn Cys Tyr Phe Pro Leu Gln Ser Tyr
                485                 490                 495
Gly Phe Gln Pro Thr Asn Gly Val Gly Tyr Gln Pro Tyr Arg Val Val
            500                 505                 510
Val Leu Ser Phe Glu Leu Leu His Ala Pro Ala Thr Val Cys Gly Pro
        515                 520                 525
Lys Lys Ser Thr Asn Leu Val Lys Asn Lys Cys Val Asn Phe Asn Phe
    530                 535                 540
Asn Gly Leu Thr Gly Thr Gly Val Leu Thr Glu Ser Asn Lys Lys Phe
545                 550                 555                 560
Leu Pro Phe Gln Gln Phe Gly Arg Asp Ile Ala Asp Thr Thr Asp Ala
                565                 570                 575
Val Arg Asp Pro Gln Thr Leu Glu Ile Leu Asp Ile Thr Pro Cys Ser
            580                 585                 590
Phe Gly Gly Val Ser Val Ile Thr Pro Gly Thr Asn Thr Ser Asn Gln
        595                 600                 605
Val Ala Val Leu Tyr Gln Asp Val Asn Cys Thr Glu Val Pro Val Ala
    610                 615                 620
Ile His Ala Asp Gln Leu Thr Pro Thr Trp Arg Val Tyr Ser Thr Gly
625                 630                 635                 640
Ser Asn Val Phe Gln Thr Arg Ala Gly Cys Leu Ile Gly Ala Glu His
                645                 650                 655
Val Asn Asn Ser Tyr Glu Cys Asp Ile Pro Ile Gly Ala Gly Ile Cys
            660                 665                 670
Ala Ser Tyr Gln Thr Gln Thr Asn Ser Pro Arg Arg Ala Arg Ser Val
        675                 680                 685
Ala Ser Gln Ser Ile Ile Ala Tyr Thr Met Ser Leu Gly Ala Glu Asn
    690                 695                 700
Ser Val Ala Tyr Ser Asn Asn Ser Ile Ala Ile Pro Thr Asn Phe Thr
705                 710                 715                 720
Ile Ser Val Thr Thr Glu Ile Leu Pro Val Ser Met Thr Lys Thr Ser
                725                 730                 735
Val Asp Cys Thr Met Tyr Ile Cys Gly Asp Ser Thr Glu Cys Ser Asn
            740                 745                 750
Leu Leu Leu Gln Tyr Gly Ser Phe Cys Thr Gln Leu Asn Arg Ala Leu
        755                 760                 765
Thr Gly Ile Ala Val Glu Gln Asp Lys Asn Thr Gln Glu Val Phe Ala
    770                 775                 780
Gln Val Lys Gln Ile Tyr Lys Thr Pro Pro Ile Lys Asp Phe Gly Gly
785                 790                 795                 800
Phe Asn Phe Ser Gln Ile Leu Pro Asp Pro Ser Lys Pro Ser Lys Arg

                    805                     810                     815
Ser Phe Ile Glu Asp Leu Leu Phe Asn Lys Val Thr Leu Ala Asp Ala
                820                     825                     830
Gly Phe Ile Lys Gln Tyr Gly Asp Cys Leu Gly Asp Ile Ala Ala Arg
            835                     840                     845
Asp Leu Ile Cys Ala Gln Lys Phe Asn Gly Leu Thr Val Leu Pro Pro
    850                     855                     860
Leu Leu Thr Asp Glu Met Ile Ala Gln Tyr Thr Ser Ala Leu Leu Ala
865                     870                     875                     880
Gly Thr Ile Thr Ser Gly Trp Thr Phe Gly Ala Gly Ala Ala Leu Gln
                885                     890                     895
Ile Pro Phe Ala Met Gln Met Ala Tyr Arg Phe Asn Gly Ile Gly Val
            900                     905                     910
Thr Gln Asn Val Leu Tyr Glu Asn Gln Lys Leu Ile Ala Asn Gln Phe
        915                     920                     925
Asn Ser Ala Ile Gly Lys Ile Gln Asp Ser Leu Ser Ser Thr Ala Ser
    930                     935                     940
Ala Leu Gly Lys Leu Gln Asp Val Val Asn Gln Asn Ala Gln Ala Leu
945                     950                     955                     960
Asn Thr Leu Val Lys Gln Leu Ser Ser Asn Phe Gly Ala Ile Ser Ser
                965                     970                     975
Val Leu Asn Asp Ile Leu Ser Arg Leu Asp Lys Val Glu Ala Glu Val
            980                     985                     990
Gln Ile Asp Arg Leu Ile Thr Gly Arg Leu Gln Ser Leu Gln Thr Tyr
        995                     1000                    1005
Val Thr Gln Gln Leu Ile Arg Ala Ala Glu Ile Arg Ala Ser Ala Asn
    1010                    1015                    1020
Leu Ala Ala Thr Lys Met Ser Glu Cys Val Leu Gly Gln Ser Lys Arg
1025                    1030                    1035                    1040
Val Asp Phe Cys Gly Lys Gly Tyr His Leu Met Ser Phe Pro Gln Ser
                1045                    1050                    1055
Ala Pro His Gly Val Val Phe Leu His Val Thr Tyr Val Pro Ala Gln
            1060                    1065                    1070
Glu Lys Asn Phe Thr Thr Ala Pro Ala Ile Cys His Asp Gly Lys Ala
        1075                    1080                    1085
His Phe Pro Arg Glu Gly Val Phe Val Ser Asn Gly Thr His Trp Phe
    1090                    1095                    1100
Val Thr Gln Arg Asn Phe Tyr Glu Pro Gln Ile Ile Thr Thr Asp Asn
1105                    1110                    1115                    1120
Thr Phe Val Ser Gly Asn Cys Asp Val Val Ile Gly Ile Val Asn Asn
                1125                    1130                    1135
Thr Val Tyr Asp Pro Leu Gln Pro Glu Leu Asp Ser Phe Lys Glu Glu
            1140                    1145                    1150
Leu Asp Lys Tyr Phe Lys Asn His Thr Ser Pro Asp Val Asp Leu Gly
        1155                    1160                    1165
Asp Ile Ser Gly Ile Asn Ala Ser Val Val Asn Ile Gln Lys Glu Ile
    1170                    1175                    1180
Asp Arg Leu Asn Glu Val Ala Lys Asn Leu Asn Glu Ser Leu Ile Asp
1185                    1190                    1195                    1200
Leu Gln Glu Leu Gly Lys Tyr Glu Gln Tyr Ile Lys Trp Pro Trp Tyr
                1205                    1210                    1215
Ile Trp Leu Gly Phe Ile Ala Gly Leu Ile Ala Ile Val Met Val Thr
            1220                    1225                    1230
Ile Met Leu Cys Cys Met Thr Ser Cys Cys Ser Cys Leu Lys Gly Cys
        1235                    1240                    1245
Cys Ser Cys Gly Ser Cys Cys Lys Phe Asp Glu Asp Asp Ser Glu Pro
    1250                    1255                    1260
Val Leu Lys Gly Val Lys Leu His Tyr Thr
1265                    1270

## Description

### Technical Field

The present invention regards new avirulent virus-like particles and their use as analytical reagents and for vaccination against viruses, in particular corona viruses.

### Background Art

Immunization against new emerging viral infections and their accurate detection (i.e. level of immunity in a person) are tightly coupled as both require agents that mimic the virus as close as possible, yet obviously are not infectious. In the case of accurate detection of the level of immunity, an analytical reagent performs best if it best matches with the agent that triggered the formation of the specific set of antibodies, since all humans have different antibodies. If an analytical reagent for detection of the level of immunity is ill matching the agent (e.g. a virus), the use of the analytical reagent will result in wrong positive and wrong negative results on the level of immunity of a person.

Interestingly enough, the same benefit of an as perfect as possible match between the analytical reagent and the agent of interest (i.e. the virus) is found in the development of a vaccine, as now the immune system of a person is to be triggered and trained as accurately as possible to later recognize again a most similar agent (i.e. the virus trying to infect the person).

For viruses, so called virus-like particles (VLP) have been developed in a great variety using a number of surrogates for the virus's core and most components from its shell. This strategy has been successful in many cases, but its overall degree of protection is limited by its quality of the match.

The mimicking action's quality depends on the molecular orientation, identity of the subunits of the structure and the overall wetting behavior of the object, its size and charge. Each factor is known per se and extensively discussed in the scientific literature.

This relationship is very important for virus types with complex surfaces, such as corona viruses, as their spike proteins protrude far into the surrounding liquid, whilst providing a complex local environment at the 1 to 10 nm resolution between the spike protein and the virus hull. Since this local microenvironment is at the same size than a typical antibody, corona viruses are notoriously difficult to target. One reason for this may be that antibodies have to interact with a mobile (i.e. conformational flexible) target, whilst being hindered in adapting a specific configuration when trying to sneak between hull components and hull spike proteins.

The so-called gold standard for a virus mimicry would be a complete outer shell of a virus, with all components identical or essentially identical to the ones of the infectious virus itself. A number of strategies have been developed over the decade, to either use existing, known viruses and modify them with a motive of the target virus (e.g. change a protein at the outer surface of the carrier), or to inactivate the virus. Later strategy appears attractive as it would provide a perfect copy of the target virus, but a non-reproductive/infective one. In theory, this sounds easy, but in practice, the inactivation is a delicate balance between the key needs:
a) Complete inactivation of the virus, as if even a single virus survives the inactivation, it may infect a person undergoing vaccination and then cause an outbreak. Hence, the quality measures are enormous, both for avoiding mixing during production, i.e. a case where some parts of the vaccine manufacturing is getting in contact with the final product, and to make sure all of the material is inactivated (batch homogeneity)
b) Keeping the inactivated virus's molecular recognition properties as identical to the target virus (infective) as possible. This is more difficult than it sounds, since either the inactivation is done through a chemical derivatization, e.g. crosslinking with formaldehyde or glutaraldehyde, or thermally or through other physico/chemical means. In all cases, the inactivation must change vital parts of the virus to a point where it is truly damaged.

In such a procedure, the inactivation must work so good that it does not miss a single virus out of 10 billion viruses, often better than not missing a single virus out of over 10 to the power of 14 viruses. Such quality requirements result in over-inactivation, i.e. the average inactivated virus is so heavily damaged that it does not anymore much resemble the original version (i.e. the target).

As a result of this, it is of high interest, to develop precise methods to prepare virus surrogates / virus mimics with a quality protocol that allows regulatory authorities and manufacturers to easily follow the preparation in a most detailed way.

The analysis of blood serum of a population is a second topic that requires the same surrogates / mimics as above. The outcome of a vaccination (successful immunization or not) is measured by the presence of antibodies in a person after a given time post vaccination. It is also of high interest to know which persons in a population have already had a given infection (i.e. they are immune or at least less prone to infection than others), or to measure their level of immunity. Since humans build a large variety of antibodies, detection either requires a large range of low quality virus surrogates or moieties displaying characteristic parts of a virus, or a test method could use a near perfect surrogate / mimic of the virus and thus identify any possible immune reaction against its (infectious) counterpart (i.e. the real virus).

There is a third, most important factor in this development, and it is linked to the often rapid mutation of such viruses over the course of an infection. Since easy to target parts of a virus' shell are rapidly fought by the human immune system, there is always a huge evolutionary pressure on the virus to mutate in directions where it is more and more elusive. As infections progress, an original virus may develop new forms (mutations) that then have better or worse infectious properties and allow the virus to better (or less) replicate. In any case, an original vaccination may then fail, as the above evolutionary pressure will guide the virus away from easy to target moieties of its shape. If new versions of a virus emerge, they will be per definition different to the earlier ones particularly when it concerns the key parts targeted in the previous vaccination strategy.

### Disclosure of the Invention

Hence, it is a general object of the invention to provide an improved virus mimicry or surrogate that will have at least the first of the following two tasks:
(i) To serve as an analytical agent to help identification of immunity (titer) in humans and /or to serve as part of a vaccination, and
(ii) To be quickly adaptable to new forms of a virus (i.e. a mutation)

Now, in order to implement these and still further objects of the invention, which will become more readily apparent as the description proceeds, the avirulent virus-like particle is manifested by the features that it comprising the spike protein S of a target corona virus and at least two and preferably 3 shell-forming proteins being further membrane proteins of corona viruses, said shell-forming proteins being the structural envelope protein E and the membrane protein M and preferably also the nucleoprotein N, said virus-like particle comprising a protein shell and a glycosylation pattern obtainable by expression in mammalian cells.

While the further membrane proteins can be proteins of corona viruses other than the target virus, in preferred embodiments of the avirulent virus-like particle the further membrane proteins are proteins of the target corona virus.

In another embodiment the target virus is selected from the group consisting of members of coronaviridae, betacoronaviruses, Sars-CoV 1, Mers-CoV and in particular Sars CoV 2.

A preferred mammalian cell is selected from the group consisting of CHO cells, like ExpiCHO, or human cell lines, like HEK903T, HT-1080, PER.C6, CAP, HuH-7 and HKB-11.

A further aspect of the present invention is a method for producing an avirulent virus-like particle as described above, wherein
(i) nucleotide sequences, in particular DNA sequences, each encoding the S-protein or one of the shell-forming proteins and optionally transcriptional and translational control elements is chemically synthesized by de novo DNA synthesis,
(ii) a mammalian cell is transfected with at least three, preferably four of said nucleotide sequences and cultured
(iii) the avirulent virus-like particles produced by said mammalian cell are harvested.

Further aspects of the present invention regard
a use of the avirulent virus-like particles as described above for analyzing antibodies against the target virus in a mammalian sample, in particular a blood sample,
a use of an avirulent virus-like particle as described above as effective substance of a vaccine,
an analytical agent comprising at least one kind of an avirulent virus-like particle as described above,
a vaccine comprising at least one kind of avirulent virus-like particles as described above
a method for producing nucleic acid sequences encoding the spike protein S of a target corona virus and at least two and preferably three shell-forming proteins being further membrane proteins of corona viruses, said shell-forming proteins being the structural envelope protein E and the membrane protein M and optionally and preferably also the nucleoprotein N, said nucleic acids optionally also comprising transcriptional and translational control elements and wherein said nucleic acids are adapted or optimized for chemical synthesis and preferably also for expression in mammalian cells, said method comprising
(i) determining short partial sequences, i.e. oligonucleotides, having unique ends and preferably a length of 50 to 100 bp
(ii) chemically synthesizing said oligonucleotides
(iii) combining two or more of said oligonucleotides to larger partial sequences and optionally repeating this step until partial sequences of desired length are obtained, this step being e.g. performed by polymerase chain assembly (PCA),
(iv) optionally combining two or more of the sequences of step (iii) to obtain the nucleic acid sequences, i.e. the polypeptides, encoding the spike protein S of the target corona virus and the shell-forming proteins, e.g. by means of enzymatic ligation or isothermal assembly.

By PCA in general oligonucleotides or polynucleotides of up to about 1000 bp length are obtainable. For lager polynucleotides, i.e. up to 4000 bp or even 10000 bp step (iv) has to be performed.

Other objects of the present invention are a transfection vector preferably a plasmid, comprising a nucleic acid sequence as described above and/or
a mammalian cell comprising the nucleic acid sequences, i.e. the polypeptides encoding the S-protein, the M-protein, the E-protein and optionally and preferably the N-protein as described above.

Step (iii) can also comprise an amplification step.

The transfection vector or plasmid can comprise all elements needed for protein expression prior to protein insertion or such elements can be introduced together with the protein encoding sequence. In case of plasmids, they can be multiplied in e.g. bacteria, like E. coli and then harvested according to known methods.

The mammalian cells can be obtained by co-transfecting them with a multitude of plasmids comprising the polynucleotides encoding the three or preferably four proteins needed for the VLP formation.

The second task indicated above is time critical. If a virus spreads, the time lag between recognizing emergence of a mutation and action in the form of providing a new vaccination, and being able to accurately detect presence of immunity / titer in the population directly translates into severity of the infection.

In an ideal case, a new vaccine could be manufactured within days of detection of a new mutation, and being distributed to people that potentially got close to the source of that new mutation. Such readily adaptable and available vaccinations are not yet realized, and cannot be done within the hitherto existing pharmaceutical knowledge and capability.

In such an understanding, both the detection and the vaccination are dynamic, i.e. for a given virus, different mutations can occur at different locations and at different times so that the detection and fight against said viral disease demands a string of reagents, an array of reagents, that may differ locally and in time. Such array (dependent of time and location) of reagents (for detection), also termed analytical agents or analytical reagents, and vaccines may be called a "dynamic detection and vaccination reagent group". In the context of this invention, this array of reagents, the knowledge on where and how to apply its components (the detection component of the array with the materially important part being the VLP, also called analytical reagent) and the corresponding vaccines (similar or even identical to the key component of the analytical reagent) are encompassed by the term "dynamic detection and vaccination reagent group".

For practical purposes, such a "dynamic detection and vaccination reagent group" may consist first of at least two reagents (typically liquid formulations containing at least one kind of VLPs, wherein each reagent has another kind or composition of VLPs, i.e. the VLPs of the different kinds differ in terms of at least the relevant protein structure and / or the folding of at least the relevant protein, in general the S-protein. The "dynamic detection and vaccination reagent group" may grow over the course of an infection, and become diverse with partially different components for a given geographic region and/or time (beginning, peak, fading out part of an epidemic).

The "dynamic detection and vaccination reagent group" may be represented by a matrix containing the individual components. For practical reasons it may be advantageous to use one axis for geography, and another one for time, but other grouping or arrays can be used. The group may also just be two or a few, like 2 to 10 reagents in the case of a small group and many, like more than 10 reagents in the case of large groups.

The "dynamic detection and vaccination reagent group" may comprise a basic composition common to all reagents, said basic composition containing the individual VLPs of each reagent.

As indicated above, in case of rapidly mutating viruses, it is important to be able to react to such mutations very fast. A suitable method for such fast reaction is a method for producing a nucleic acid sequence encoding a mutated protein, in general the spike protein S of a target corona virus that bears said at least one mutation, said method comprising (provided that the VLP against the original virus has already been synthesized as described above and partial sequences are available from said synthesis)
(i) chemically synthesizing one or more short partial sequence, oligonucleotides, each comprising at least one mutation,
(ii) combining said one or more oligonucleotides comprising said mutations with each other and/or with at least one further short partial sequence to obtain a larger mutated partial sequence, optionally repeating this step until a partial sequence of desired length is obtained, e.g. via polymerase chain assembly (PCA)
(iii) optionally combining said larger mutated partial sequences with each other and/or with at least one larger partial sequence to obtain the nucleic acid sequence, the polypeptide, encoding the mutated protein, in particular the spike protein S of the mutated target corona virus.

Step (iii) is performed in case of large polynucleotides, i.e. polynucleotides of more than e.g. 1000 bp.

In an alternative embodiment that is especially suitable if only few, like one nucleotide have/has mutated, instead of chemical synthesis site-directed mutagenesis can be applied in any of the above indicated steps.

Also a transfection vector comprising such a mutated nucleic acid sequence and/or a mammalian cell comprising such mutated nucleic acid sequence besides of polynucleotides encoding other, in general not mutated proteins needed for VLP production are further aspects of the present invention. Such vectors/plasmids and mammalian cells, besides of the mutations, are as described above.

An avirulent VLP directed to the original virus or an avirulent VLP directed to a mutation can be the active substance of an analytical agent / reagent or of a vaccine. In a further embodiment, the analytical agent / reagent or the vaccine comprises a combination of at least a first and a second avirulent virus-like particle, said first and said second avirulent virus-like particles having S-proteins of the original virus and a mutation or the S-proteins of different mutations of one and the same original virus, said original virus and said mutation or said two mutations differing in at least one different amino acid and/or at least one deletion and/or at least one insertion of an amino-acid residue,

At least two analytical agents / reagents and/or at least two vaccines are the components of a "dynamic detection and vaccination reagent group".

The analytical agents / reagents or the vaccine contain solvents, additives, preservatives, adjuvants and other components, or/and - in the case of analytical agents /re-agents - binding agents to link the VLP to analytical equipment, sample holders, plates etc. Suitable components for formulations of analytical agents and /or vaccines are known to the skilled person.

Since suitable non-VLP materials are known, the non-VLP materials are of less interest in this invention and it shall be understood that two components of a "dynamic detection and vaccination reagent group" shall be considered identical if their VLP(s) are identical with regard to their sequence and functionality. For clarity, two components of a "dynamic detection and vaccination reagent group" shall be considered identical even if their non-VLPs are different or if different amounts or types of such non-VLP materials are contained.

A dynamic detection and vaccination reagent group is different from a vaccine, or an analytical assay for a given detection task in that
(a) it is a group of reagents or vaccines, meaning that there are at least two such reagents or vaccines, and for analysis or vaccination only part of said reagents or vaccines is used at a time and a geographic location,
(b) the members of the group may change over time, and they are depending on geographic location and time, and - preferably -
(c) at least one member of the group is both useful for the detection of antibodies in a population and it is useful as a component in the manufacturing of a corresponding vaccine,
(d) the group's size and composition reacts on mutations and changes in the virus in that its size and the composition of the analytical agents /reagents and/or vaccines it comprises can be rapidly adapted,
(e) the members of the group are similar to one another as they are used against the same type of virus derived from the same original infection (onset of a spread of infections), and this similarity can be recognized through the similarity of large parts of the coded proteins.

Typical changes and modifications in a protein as a result of a mutation in a virus are known to the person skilled in the art. Due to this dynamic, the same "dynamic detection and vaccination reagent group" shall be assumed to still be present if one or more member(s) of such reagent group or vaccination is/are changed as a result of a mutation.

In one embodiment, the present invention describes a VLP with two applications. These are: First, the VLP can be used for the detection of the level of immunity against a coronavirus in a typical immunoassay. The reagent excels against other, less precise reagents, in terms of specificity and the lower level of false negative or false positive results. Second, the essentially same VLP is useful in the preparation of a vaccine against the same virus.

The inventive avirulent VLP differs from previous attempts to develop similar VLP in that
(i) it has been obtained starting from molecularly defined entities that comprise the nucleotide sequence encoding the spike or S-protein and the nucleotide sequences encoding at least two, preferably three further shell proteins, i.e. the structural envelope or E-protein and the membrane or M-protein and optionally and preferably also the nucleoprotein or N-protein, said nucleotide sequences being adapted/optimized for chemical synthesis and preferably also for protein expression in mammalian cells, and
(ii) said VLP having been produced in mammalian cells.

In one of the embodiments, the present invention describes a group of two VLPs that are similar to one another, but represent an original virus and a first mutation of it. Both reagents of this group can be used for the detection of the level of immunity against a specific coronavirus in a typical immunoassay. It is obviously advantageous to then report the immunity against the original and the mutated virus. In an alternative embodiment, the two VLPs can be present in one and the same reagent. In such case, whether only the information about immunity or not or the information against which virus is obtainable depends on the distinguishability or the labelling of the VLPs, respectively.

In the context of this invention, a mutation is a change in one or several nucleic acids in the coding sequence (genome level view) resulting in a change of one or several amino acids (in the expression level view). A mutation can be a single point mutation (single nucleotide changed, affording a change of one amino acid in the expressed protein), or multiple changes, but limited to nucleotide changes resulting in a maximum of 20 % of all amino acids, if the mutation is a naturally occurring one, or limited to mutations that change the original proteins form or function in a most essential way, if the mutation is an artificially generated sequence, since e.g. computationally generated sequences can be changed over larger areas, while essentially keeping the function of the protein.

As already indicated above, in yet a further embodiment, the present invention describes an immunoassay that uses VLPs as an analytical agent / reagent. The details of attaching such reagent to a suitable sample handling system, e.g. a modified polystyrene plate, is well known to the person skilled in the art. In an alternative embodiment, the inventive VLPs may also be used free flowing in a solution, e.g. when using a metal nanoparticle based color assay.

The immunoassay may often be commercially used years or months ahead of a potential use as vaccine, since the regulatory approval process for vaccines typically takes years. The delayed use as vaccine does not preclude, however, the early use of the inventive VLPs for the detection of a level of immunity.

In yet another embodiment, also already addressed above, the present invention provides a "dynamic detection and vaccination reagent group" to fight a broadly progressing infection with various subtypes of a virus, or mutations. Such subtypes or mutations may be present geographically (i.e. at the same time appearing at different locations), or sequentially (i.e. at different times, appearing at the same location). For practical reasons, the group is started as a small group if a new virus is detected, and grows over time, when a virus adapts and changes.

### Brief Description of the Drawings

The invention will be better understood and objects other than those set forth above will become apparent when consideration is given to the following detailed description thereof. Such description makes reference to the annexed drawings, wherein:
Figure 1 is a transmission electron microscopy (TEM) image of a VLP after staining with uranylacetate on carbon-coated cooper grids.
Figure 2 is a TEM image showing goat anti-mouse antibodies coupled to gold nanoparticles detecting a primary mouse antibody recognizing the Sars-2 Spike-protein.

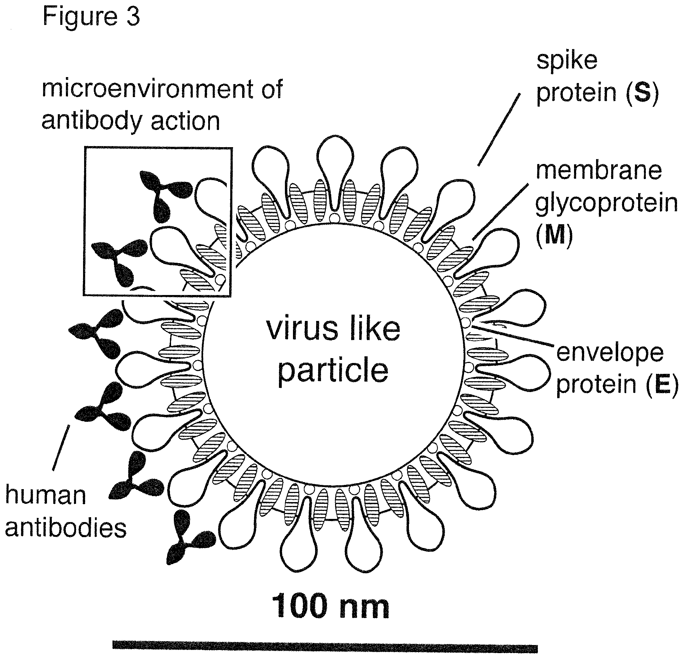

Figure 3 shows the microenvironment of antibody action.

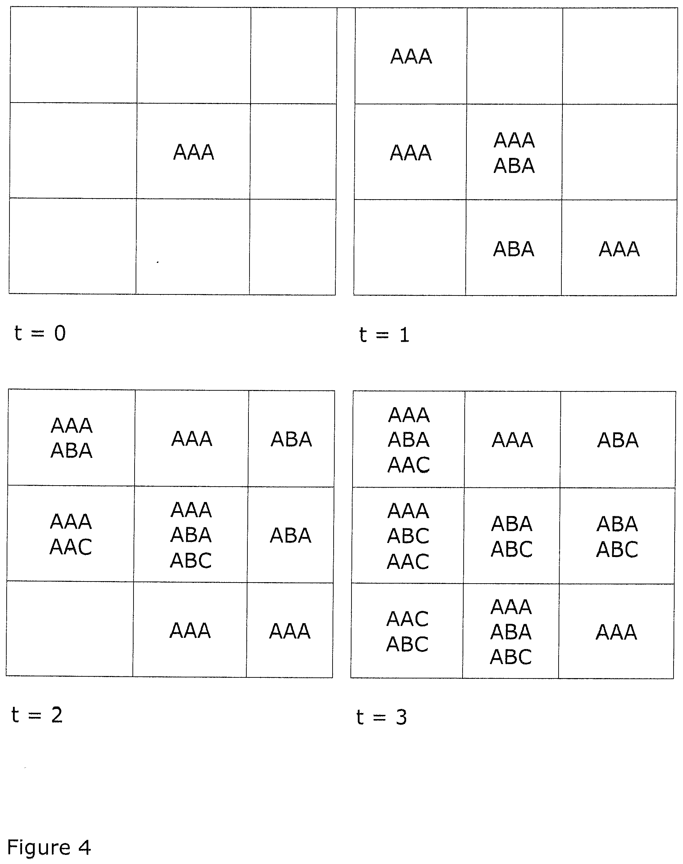

Figure 4 schematically shows the development of virus mutations and respectively the dynamic detection and vaccination reagent group.

### Modes for Carrying Out the Invention

In one embodiment of the present invention, the VLPs are further characterized by the following features:

### Size:

The diameter of avirulent VLP particles of the present invention is on average 105 nanometer with a standard-deviation of 21 nanometer. (The actual data found are 105.31 and +/- 21.30 nm as standard deviation.)

### Composition:

The VLPs of the present invention consist of spherical vesicles composed of an inner aqueous phase enclosed by a membrane envelope.
- The aqueous phase comprises the N protein as sole protein- if any - and no virus RNA or DNA.
- The membrane envelope consists of a lipid bilayer in which the integral membrane glycoproteins, the M-protein, the S-protein and the E-protein are embedded to form the membrane envelope portion of the VLPs. Whereby:
- The M-protein is the most abundant structural protein component of the VLP envelope (with more than 50% of the membrane surface covered by the M-protein).
- The S-protein is the second most abundant component of the VLP envelope and
- The E-protein is a minor structural protein component (with less than 10% of the membrane surface covered by the E-protein).

### Posttranslational modifications like glycosylation:

The membrane protein components are further characterized by the following N-glycosylation pattern at distinct asparagine residues characterized by Endoglycosidase H and Peptide-N-glycosidase F sensitive high-mannose N-glycans, afucosylated and fucosylated hybrid-type glycans and glycans from the complex type.

The following amino acid positions refer to a DNA construct with one additional amino acid inserted directly after the start codon.
- The M-protein is predominately N-glycosylated at five N-linked glycan sites annotated by the amino acid positions (N6), (N22), (N44), (N122) and (N204).
- The E protein is predominately N-glycosylated at two N-linked glycan sites annotated by the amino acid positions (N49) and (N67).
- The S protein is N-glycosylated at twenty two N-linked glycan sites annotated by the amino acid positions (N18), (N62), (N75), (N123), (N150), (N166), (N235), (N283), (N332), (N344), (N604), (N617), (N658), (N710), (N718), (N802), (N1075), (N1099), (N1135), (N1159), (N1174) and (N1195).

The protein sequences of a Sars-2-CoV that has been found in an early stage of the pandemic and that can be viewed as original sequences but with an added valine directly after the start methionine for improved translation are as follows:
Envelope protein E 
MVYSFVSEETGTLIVNSVLLFLAFVVFLLVTLAILTALRLCAYCCNIVNVSLVKPSFYVYSRVKNLNS
SRVPDLLV*
Membrane protein M 
MVADSNGTITVEELKKLLEQWNLVIGFLFLTWICLLQFAYANRNRFLYIIKLIFLWLLWPVTLACF
VLAAVYRINWITGGIAIAMACLVGLMWLSYFIASFRLFARTRSMWSFNPETNILLNVPLHGTILT
RPLLESELVIGAVILRGHLRIAGHHLGRCDIKDLPKEITVATSRTLSYYKLGASQRVAGDSGFAAYS
RYRIGNYKLNTDHSSSSDNIALLVQ*
Nucleoprotein 
MVSDNGPQNQRNAPRITFGGPSDSTGSNQNGERSGARSKQRRPQGLPNNTASWFTALTQH
GKEDLKFPRGQGVPINTNSSPDDQIGYYRRATRRIRGGDGKMKDLSPRWYFYYLGTGPEAGLP
YGANKDGIIWVATEGALNTPKDHIGTRNPANNAAIVLQLPQGTTLPKGFYAEGSRGGSQASSR
SSSRSRNSSRNSTPGSSRGTSPARMAGNGGDAALALLLLDRLNQLESKMSGKGQQQQGQTVT
KKSAAEASKKPRQKRTATKAYNVTQAFGRRGPEQTQGNFGDQELIRQGTDYKHWPQIAQFAP
SASAFFGMSRIGMEVTPSGTWLTYTGAIKLDDKDPNFKDQVILLNKHIDAYKTFPPTEPKKDKKK
KADETQALPQRQKKQQTVTLLPAADLDDFSKQLQQSMSSADSTQA*
Surface proteins S

MVFVFLVLLPLVSSQCVNLTTRTQLPPAYTNSFTRGVYYPDKVFRSSVLHSTQDLFLPFFSNVTW
FHAIHVSGTNGTKRFDNPVLPFNDGVYFASTEKSNIIRGWIFGTTLDSKTQSLLIVNNATNVVIKV
CEFQFCNDPFLGVYYHKNNKSWMESEFRVYSSANNCTFEYVSQPFLMDLEGKQGNFKNLREF
VFKNIDGYFKIYSKHTPINLVRDLPQGFSALEPLVDLPIGINITRFQTLLALHRSYLTPGDSSSGWT
AGAAAYYVGYLQPRTFLLKYNENGTITDAVDCALDPLSETKCTLKSFTVEKGIYQTSNFRVQPTES
IVRFPNITNLCPFGEVFNATRFASVYAWNRKRISNCVADYSVLYNSASFSTFKCYGVSPTKLNDLC
FTNVYADSFVIRGDEVRQIAPGQTGKIADYNYKLPDDFTGCVIAWNSNNLDSKVGGNYNYLYRL
FRKSNLKPFERDISTEIYQAGSTPCNGVEGFNCYFPLQSYGFQPTNGVGYQPYRVVVLSFELLHA
PATVCGPKKSTNLVKNKCVNFNFNGLTGTGVLTESNKKFLPFQQFGRDIADTTDAVRDPQTLEI
LDITPCSFGGVSVITPGTNTSNQVAVLYQDVNCTEVPVAIHADQLTPTWRVYSTGSNVFQTRA
GCLIGAEHVNNSYECDIPIGAGICASYQTQTNSPRRARSVASQSIIAYTMSLGAENSVAYSNNSIA

IPTNFTISVTTEILPVSMTKTSVDCTMYICGDSTECSNLLLQYGSFCTQLNRALTGIAVEQDKNTQ
EVFAQVKQIYKTPPIKDFGGFNFSQILPDPSKPSKRSFIEDLLFNKVTLADAGFIKQYGDCLGDIAA
RDLICAQKFNGLTVLPPLLTDEMIAQYTSALLAGTITSGWTFGAGAALQIPFAMQMAYRFNGIG
VTQNVLYENQKLIANQFNSAIGKIQDSLSSTASALGKLQDVVNQNAQALNTLVKQLSSNFGAIS
SVLNDILSRLDKVEAEVQIDRLITGRLQSLQTYVTQQLIRAAEIRASANLAATKMSECVLGQSKRV
DFCGKGYHLMSFPQSAPHGVVFLHVTYVPAQEKNFTTAPAICHDGKAHFPREGVFVSNGTHW
FVTQRNFYEPQIITTDNTFVSGNCDVVIGIVNNTVYDPLQPELDSFKEELDKYFKNHTSPDVDLG
DISGINASVVNIQKEIDRLNEVAKNLNESLIDLQELGKYEQYIKWPWYIWLGFIAGLIAIVMVTIM
LCCMTSCCSCLKGCCSCGSCCKFDEDDSEPVLKGVKLHYT*

As already indicated above, the VLPs are preferably produced by a method involving the chemical synthesis of the protein encoding polynucleotides. For fast chemical synthesis the protein sequence is "reverse-translated" into the coding sequence in a manner optimized for chemical synthesis and preferably also for expression in mammalian cells. This means that in a first step the DNA or RNA sequence is reduced in repetitive nucleotides or short repetitive sequences at least in putative end regions of oligonucleotides using the degeneration of the genetic code. Simultaneously or subsequently oligonucleotides of about 50 to 100 bp length are determined having unambiguous ends so that connection of such oligonucleotides, e.g. via PCA, results in one predetermined polynucleotide sequence. This step can be repeated until the oligonucleotides or polynucleotides of desired length are obtained. PCR can be used to amplify the nucleotide sequences until the desired number of nucleotide sequences of desired length are obtained.

The oligonucleotide or polynucleotide sequence obtainable via PCA in general is a sequence of about 1000 bp length. Longer polynucleotides are obtainable via enzymatic reaction, e.g. by ligases, or via isothermal assembly provided that the sticky ends of the short polynucleotides are suitable. Via enzymatic ligation or isothermal assembly long polynucleotides of 4000 to 10000 bp length are obtainable. The optimization of the polynucleotide sequence for chemical synthesis can be done by means of a computer. Firms offering such service and even the synthesized polynucleotide exist. One of them is Gigabases AG, Switzerland.

Once the full length polynucleotides, each encoding one of the proteins, are synthesized, they are introduced into expression plasmids or vectors suitable for protein expression in mammalian cells. The plasmids can be multiplied in e.g. bacterial cells like E. coli.

Since mammalian cells are able to take up a multitude of plasmids, i.e. up to 50 or even up to 100 plasmids, mammalian cells can be co-transfected with a mixture of the plasmids for the expression of at least three, preferably all four proteins. Co-transfection with plasmids encoding three or four different proteins has proved possible since it was found that the VLPs are self-assembling in the "correct" manner irrespective of the number of plasmids for a specific protein in a cell as long as the polynucleotides for all necessary proteins are present.

This self-assembling provides the further advantage that the VLP production can be accelerated by optimizing the plasmid composition so that most cells get the number of plasmids of each kind that is needed for expression of each of the proteins in the needed amount.

If one or more mutations occur, the oligonucleotides or polynucleotide encoding the full mutated protein or parts thereof can either be adapted via site-directed mutagenesis or - in particular if many mutations should simultaneously be introduced - via new synthesis of the respective one or more oligonucleotides or polynucleotides.

While a full synthesis of a long protein today may still take up to about 5 weeks, it is expected that the technique improves over the next years so that such synthesis will only take a few days. Already today the reaction on a mutation can be fast, provided that different lengths of oligonucleotides and short polynucleotides are kept on stock.

### Examples.

### Example 1. Preparation of a VLP.

A synthetic nucleic acid sequence containing the protein-coding sequences for the expression of the structural envelope protein E, the membrane protein M and the nucleoprotein N from Corona virus Sars-2 or MHVA59 and the surface protein S of Sars-2-CoV were sequence optimized for chemical synthesis and ordered from a long-chain oligonucleotide manufacturer (Gigabases AG, Switzerland). Protein-coding sequences were PCR amplified from synthetic DNA templates using oligo-nucleotide primers that added flanking restriction enzyme sites and appropriate translation initiation signals, corresponding to the consensus Kozak sequence upstream of start codons. PCR amplification was performed with Phusion polymerase (New England Biolabs). PCR products were separated on agarose-gel electrophoresis, and products of the correct size were isolated and purified over a Nucleo-Spin Column (Macherey-Nagel). Purified PCR products were subsequently cloned into mammalian expression vectors pCi and pcDNA3.4 under control of the SV40 promoter or CMV promoter. Cloning of PCR insert into linearized pCi vector was carried out by isothermal assembly using 100 ng of PCR amplicons and 100 ng of vector DNA. Insert and vector DNA was incubated with T5-exonuclease, Taq-ligase and Phusion polymerase (New England Biolabs) for one hour at 50 ºC. To clone PCR products into pcDNA3.4 vector, terminal A-overhangs were added to amplicons using Taq polymerase prior performing a Topo-TA cloning reaction (Topo-TA pcDNA3.4 Kit, Thermo Fisher). Plasmids were electro-porated into *Escherichia coli* and transformants were selected on Lurian-Broth (LB) agar plates supplemented with ampicillin. Plasmids were isolated from single colonies and sequence verified by Sanger-Sequencing. Expression plasmid series for MHV and Sars2 proteins were purified using the plasmid Midi-prep Kit (Macherey Nagel) to produce VLP in cell-lines through co-expression of the E, M, N and S-protein.

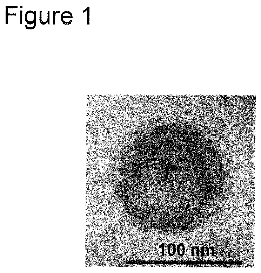

The said synthetic nucleic acid molecules were introduced into a mammalian expression cell-line ExpiCHO-S (6 x 106 cells per ml) by co-transfection of purified plasmids using ExpiFectamine CHO Transfection kit (Thermo LTC). ExpiCHO-S cells were cultivated in ExpiCHO expression medium (supplemented with 100µg/ml ampicillin and 100µg/ml streptomycine) at 37 ºC, 8% CO2 for 5-7 days on an orbital shaker, in 125ml cell-culture flask (Corning, No. 431405) with an initial culture volume of 25ml. After 18-22 h post transfection, 6ml of the ExpiCHO Feed (Thermo, LTC) and 150ul of ExpiCHO Enhancer (Thermo, LTC) was added to the cell cultures. The cell-culture supernatants were harvested and cleared from cells through a low spin centrifugation step. VLPs were then harvested from the cleared supernatant and VLP production was confirmed by transmission electron microscopy (TEM) after staining with uranylacetate on carbon-coated cooper grids and imaging on a TEM instrument. VLP of 100nm diameter were observed and confirmed by TEM (Figure 1).

### Example 2. Electron microscopic analysis of VLPs to detect VLP displaying the Sars-2-CoV S-protein.

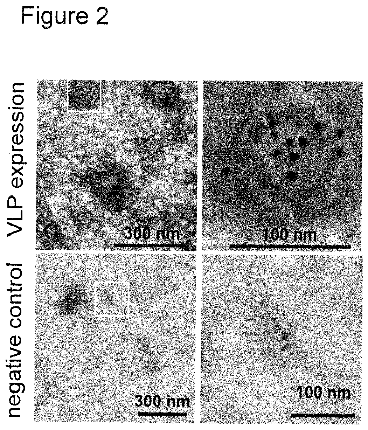

Goat anti-mouse antibodies coupled to gold nanoparticles (A31561, Invitrogen) were used to detect a primary mouse antibody recognizing the Sars-2 Spike-protein (MBS 434247, MyBioSource Inc.) through scanning transmission electron microscopy. Carbon-coated copper-mesh grids (Piano EM) were treated with poly-L-Lysine (0.01%, sterile filtered, Sigma Aldrich) for 10 min. After washing with distilled H₂O, the grids were floated on 5 µl drops of cleared cell-culture supernatant containing VLPs for 20 min. The grids were washed in phosphate buffered saline (PBS, pH=7.4, LTC) three times and incubated with a monoclonal mouse antibody against the Sars-2-CoV Spike Protein (MBS 434247, MyBioSource Inc.) for 1 hour. For the negative control, PBS was used instead of the antibody. After washing with PBS four times, the grids were incubated for 1 hour with a gold labeled secondary Goat antibody against mouse (IgG, H+L) coupled to Alexa Fluor® 488 and labeled with 10 nm colloidal gold (Invitrogen). After further washing three times in PBS, the samples were fixed in 2.5 % Glutaraldehyde (Sigma Aldrich) in PBS for 15 min, washed three times in distilled H2O and subsequently stained with 1% phosphotungstic acid (pH=7, sterile filtered, Sigma Aldrich) for 1 min. After air drying of the grids, the samples were examined using scanning transmission electron microscopy (STEM, FEI, Nova NanoSEM 450). Pictures were taken at 29 kV. VLP particles of 100 nm diameter were found to be specifically decorated with the high density 10 nm Au particles (Figure 2) demonstrating presence of the Sars-2-CoV S-protein on the surface of VLPs.

While there are shown and described presently preferred embodiments of the invention, it is to be distinctly understood that the invention is not limited thereto but may be otherwise variously embodied and practiced within the scope of the following claims.

## Claims

1. An avirulent virus-like particle comprising the spike protein S of a target corona virus and at least two and preferably 3 shell-forming proteins being further membrane proteins of corona viruses, said shell-forming proteins being the structural envelope protein E and the membrane protein M and optionally and preferably also the nucleoprotein N, said virus-like particle comprising a protein shell and a glycosylation pattern obtainable by expression in mammalian cells.

2. The avirulent virus-like particle of claim 1, wherein the further membrane proteins are proteins of the target corona virus.

3. The avirulent virus-like particle of claim 1 or 2, wherein the target virus is selected from the group consisting of members of coronaviridae, betacoronaviruses, Sars-CoV 1, Mers-CoV and in particular Sars-CoV 2.

4. The avirulent virus-like particle of any one of the preceding claims, wherein the mammalian cell is selected from the group consisting of CHO cells, like ExpiCHO, or human cell lines HEK903T, HT-1080, PER.C6,CAP, HuH-7 and HKB-11

5. A method for producing an avirulent virus-like particle of any one of claims 1 to 4, wherein
(i) nucleotide sequences, in particular DNA sequences, each encoding the S-protein or one of the shell-forming proteins and optionally transcriptional and translational control elements is chemically synthesized by de novo DNA synthesis,
(ii) a mammalian cell is transfected with at least three, preferably four of said nucleotide sequences and cultured
(iii) the avirulent virus-like particles produced by said mammalian cell are harvested.

6. Use of the avirulent virus-like particles of any one of claims 1 to 4 for analyzing antibodies against the target virus in a mammalian sample, in particular a blood sample.

7. Use of an avirulent virus-like particle of any one of claims 1 to 4 as effective substance of a vaccine.

8. An analytical agent comprising at least one kind of an avirulent virus-like particle of any one of claims 1 to 4.

9. The analytical agent of claim 8 comprising a combination of at least a first and a second avirulent virus-like particle, said first and said second avirulent virus-like particles having S-proteins of the original virus and a mutation or S-proteins of different mutations of one and the same original virus, said original virus and said mutation or said two mutations differing in at least one different amino acid and/or at least one deletion and/or at least one insertion of an amino-acid residue.

10. A vaccine comprising at least one kind of avirulent virus-like particles of any one of claims 1 to 4.

11. The vaccine of claim 10 comprising a combination of at least a first and a second avirulent virus-like particle, said first and said second virus-like particles having S-proteins of the original virus and a mutation or S-proteins of different mutations of one and the same original virus, said original virus and said mutation or said two mutations differing in at least one different amino acid and/or at least one deletion and/or at least one insertion of an amino-acid residue.

12. A method for producing nucleic acid sequences encoding the spike protein S of a target corona virus and at least two and preferably three shell-forming proteins being further membrane proteins of corona viruses, said shell-forming proteins being the structural envelope protein E and the membrane protein M and optionally and preferably also the nucleoprotein N, said nucleic acids optionally also comprising transcriptional and translational control elements and wherein said nucleic acids are adapted or optimized for chemical synthesis and preferably also for expression in mammalian cells, said method comprising
(i) determining short partial sequences, i.e. oligonucleotides, having unique ends and preferably a length of 50 to 100 bp
(ii) chemically synthesizing said oligonucleotides
(iii) combining two or more of said oligonucleotides to larger partial sequences and optionally repeating this step until partial sequences of desired length are obtained, this step being e.g. performed by PCA,
(iv) optionally combining two or more of the sequences of step (iii) to obtain the nucleic acid sequences, i.e. the polynucleotides encoding the spike protein S of the target corona virus and the shell-forming proteins, e.g. by means of enzymatic ligation or isothermal assembly.

13. A method for producing a nucleic acid sequence encoding the spike protein S of a target corona virus that is a mutation, said method comprising
(i) chemically synthesizing one or more short partial sequences, oligonucleotides, each comprising at least one mutation,
(ii) combining said one or more oligonucleotides comprising said mutations with each other and/or with at least one further short partial sequence to obtain a larger mutated partial sequence, optionally repeating this step until a partial sequence of desired length is obtained, e.g. via PCA
(iii) optionally combining said larger mutated partial sequences with each other or with at least one larger partial sequence to obtain the nucleic acid sequence, the polypeptide encoding the mutated protein, in particular the spike protein S of the mutated target corona virus .

14. A polynucleotide obtainable by the method of claim 12 or 13.

15. A transfection vector or plasmid for protein expression in mammalian cells comprising a polynucleotide of claim 14.

16. A mammalian cell comprising a polynucleotide of claim 14 or a vector or plasmid of claim 15.

17. A dynamic detection and vaccination reagent group comprising at least two analytical agents/reagents and /or at least two vaccines, each of the agents/reagents and/or vaccines comprising at least one kind of VLPs, said VLPs being dependent on geographical location and/or time.
